# EUROPEAN PATENT APPLICATION

(11) **EP 4 722 159 A1**
(43) Date of publication of application: **08.04.2026**
(21) Application number: 24815800.8
(22) Date of filing: 24.05.2024
(51) Int. Cl.: C01F 7/784, A61K 8/26

(54) **METHOD FOR PREPARING HYDRANGEA-SHAPED LAYERED DOUBLE HYDROXIDE**

(30) Priority: 26.05.2023 KR 20230068236
(71) Applicant: H&A Pharmachem Co., Ltd, Bucheon-si, Gyeonggi-do 14558 (KR)
(72) Inventor: JI, Hong Geun, Bucheon-si Gyeonggi-do 14600 (KR); PARK, Young Ah, Incheon 21069 (KR); KANG, Yu Jin, Suwon-si Gyeonggi-do 16365 (KR); SON, Seung Yeon, Bucheon-si Gyeonggi-do 14591 (KR)
(74) Representative: Schön, Christoph
(86) International application number: PCT/KR2024/007081
(87) International publication number: WO 2024/248424

(57) **Abstract**

The present invention relates to a method for preparing hydrangea-shaped layered double hydroxide and, more specifically, to a method for preparing hydrangea-shaped layered double hydroxide having a wide specific surface area by a co-precipitation method in which a NaOH solution and a mixed solution of a solution containing divalent metal cations and a solution containing trivalent metal cations are added to a solution containing anions, maintaining the pH at 9 to 11.

## Description

### TECHNICAL FIELD

The present invention relates to a method for preparing a hydrangea-shaped layered double hydroxide. More specifically, the present invention relates to a method for preparing a hydrangea-shaped layered double hydroxide having a large specific surface area by a co-precipitation method in which a mixed solution of a solution comprising a divalent metal cation and a solution comprising a trivalent metal cation, and a NaOH solution are added to a solution comprising an anion while maintaining pH at 9 to 11.

### BACKGROUND ART

Various encapsulation technologies are being studied to provide greater stability to unstable cosmetic raw materials. Specifically, it is well known that light, heat and oxygen in the air can severely reduce the biological activity of functional raw materials.

As one of such cosmetic active ingredient delivery systems, layered double hydroxide (LDH) is attracting attention. The layered double hydroxide is an inorganic compound with a layered structure, and the shape is layered like the lamellae of the stratum. In general, the composition of layered double hydroxides is expressed by the formula [M²⁺₁₋ₓ M³⁺ₓ(OH)₂]^{x+}(Aⁿ⁻)_{x/n}·mH₂O. In the formula, M²⁺ and M³⁺ are metal cations which locate octahedral sites of brucite, and Aⁿ⁻ represents an anion located between layers. Generally, in a fixed-composition phase, 0.2 ≤ x ≤ 0.33, n is 1 or 2, and m is 0.5 to 4.

LDH is basically an inorganic material with structural properties capable of absorbing various anions from small gas molecules such as carbon dioxide to biopolymers such as DNA. In addition, molecules intercalated in the layer can be kept energetically stable by electrostatic interaction with the inorganic layer. Anions have the characteristic of absorbing or releasing by ion exchange. Since the inorganic layer is composed of various compositions ranging from alkali metals to transition metals, it can be used as a precursor material for catalysts, magnetic materials, electronic materials and the like. Recently, a mechanism for self-assembling LDH nanomaterials by the use of specific molecular linkages or a mechanism for controlling the release of anions in the layer in a specific environment by selectively inducing the surface reaction of the inorganic layer has been discovered, so that it is also attracting attention as a sensor or drug delivery material. Specifically, if the electrostatic attraction existing between the layers is chemically minimized, it can be separated into a 1-nm thick nanolayer. This has become a priming powder that explosively increases the applicability of LDH materials, and LDH has been applied to fields such as nanoelectronics, magento-optics, nanosensor, etc. In addition, the LDH nanolayer-which has excellent adsorption capacity for functional organic molecules-is attracting attention as a new bio-encapsulation material based on the development of technologies such as improving the adsorption capacity of cosmetic active ingredients and controlling the release according to chemical environment control. As an example of method for preparing LDH, Korean Patent Application Publication No. 10-2019-0019150 discloses a preparation method for conferring high surface area and pore volume properties to LDH, including a specific solvent treatment step during preparing.

### [Prior Document]

### [Patent Document]

Korean Patent Application Publication No. 10-2019-0019150

### DISCLOSURE OF INVENTION

### TECHNICAL PROBLEM

Accordingly, the technical problem of the present invention is the provision of a method for preparing a layered double hydroxide having the above-described advantages in a form with a large specific surface area, thereby enabling various functional cosmetic ingredients to be efficiently delivered into the skin in a more stable state.

In addition, another technical problem of the present invention is the provision of a cosmetic composition comprising a layered double hydroxide prepared according to the above method and an active ingredient.

### SOLUTION TO PROBLEM

To solve the above technical problem, the present invention provides a method for preparing a layered double hydroxide having a large specific surface area by a co-precipitation method in which a mixed solution of a solution comprising a divalent metal cation and a solution comprising a trivalent metal cation, and a NaOH solution are added to a solution comprising an anion while maintaining pH at 9 to 11.

In addition, the present invention provides a cosmetic composition comprising a layered double hydroxide prepared according to the above method and an active ingredient.

The present invention is described in detail hereinafter.

According to one aspect to the present invention, there is provided a method for preparing a hydrangea-shaped layered double hydroxide comprising:
i) adding a mixed solution of a solution comprising a divalent metal cation and a solution comprising a trivalent metal cation, and a NaOH solution to a solution comprising an anion, while maintaining pH at 9 to 11;
ii) stirring the mixed solution obtained in step (i);
iii) filtering the resultant obtained in step (ii) through a Buchner funnel and washing with distilled water until pH reaches 6 to 8;
iv) adding a solvent to the resultant collected at the top of the Buchner funnel obtained in step (iii) and stirring; and
v) filtering the resultant obtained in step (iv) through a Buchner funnel and drying.

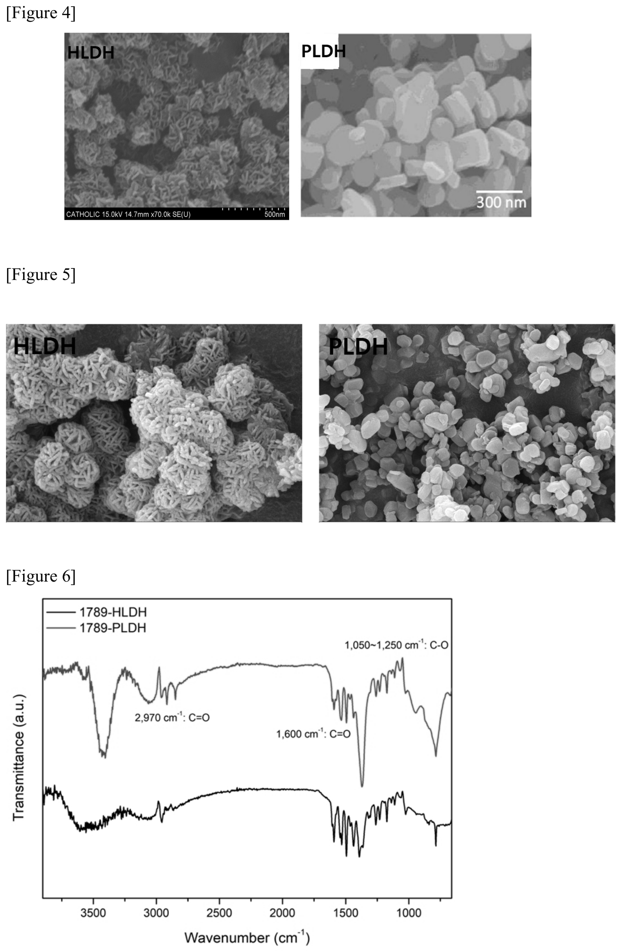

The layered double hydroxide prepared according to the preparation method of the present invention can provide a large specific surface area in the shape of hydrangea flowers (see Figures 4 and 5), and is therefore referred to herein as a "hydrangea-shaped layered double hydroxide."

In one embodiment according to the present invention, the divalent metal cation of step (i) may be Ca²⁺, Mg²⁺, Zn²⁺, Ni²⁺, Mn²⁺, Co²⁺ or Fe²⁺, but is not limited thereto.

In one embodiment according to the present invention, the trivalent metal cation of step (i) may be Al³⁺, Cr⁺, Mn³⁺, Fe³⁺, Ga³⁺, Co³⁺ or Ni³⁺, but is not limited thereto.

In one embodiment according to the present invention, the anion of step (i) may be OH⁻, F⁻, Cl⁻, Br⁻, I⁻, NO₃⁻, CO₃²⁻ or SO₄²⁻, but is not limited thereto.

According to one embodiment of the present invention, in step (i), NaOH may be used at a concentration of 2 to 5 M, 3 to 5 M, 3.5 to 4.5 M, or 4 M.

According to one embodiment of the present invention, in step (i), the pH of the solution comprising the anion may be maintained at 9.2 to 10.8, or 10 ± 0.5.

According to one embodiment of the present invention, in step (i), the mixed solution of the solution comprising the divalent metal cation and the solution comprising the trivalent metal cation, and the NaOH solution may be added to the solution comprising the anion at a rate of 1 to 4 mL/min, 1.2 to 3 mL/min, or 1.5 to 2.5 mL/min.

According to one embodiment of the present invention, in step (ii), the mixed solution may be stirred for 30 minutes to 2 hours, 40 minutes to 1.5 hours, or 1 hour.

According to one embodiment of the present invention, in step (iii), the solution may be filtered using a Buchner funnel and then washed with distilled water until the pH reaches 6 to 8, or 7.

According to one embodiment of the present invention, in step (iii), the solution may be washed with distilled water until the pH reaches 6 to 8, and then further washed with ethanol.

In one embodiment according to the present invention, the solvent of step (iv) may be ethanol.

According to one embodiment of the present invention, in step (iv), the stirring may be carried out for 12 to 36 hours, 16 to 32 hours, or 20 to 28 hours.

According to one embodiment of the present invention, the drying in step (v) may be carried out by using a vacuum chamber at room temperature.

In one embodiment according to the present invention, the hydrangea-shaped layered double hydroxide prepared by the above method may have a BET surface area of 200 m²/g or more, 210 m²/g or more, 220 m²/g or more, 230 m²/g or more, 240 m²/g or more, 250 m²/g or more, 260 m²/g or more, 270 m²/g or more, or 280 m²/g or more. In one embodiment according to the present invention, the hydrangea-shaped layered double hydroxide prepared by the above method may have a BET surface area of, for example, 250 to 300 m²/g.

According to another aspect to the present invention, there is provided a cosmetic composition comprising a hydrangea-shaped layered double hydroxide prepared by the above method according to the present invention, and an active ingredient.

In one embodiment according to the present invention, examples of the active ingredient include, but are not limited to, one or more selected from the group consisting of a moisturizer, a whitening agent, an anti-wrinkle agent, a UV blocking agent, a hair growth promoter, vitamin or a derivative thereof, amino acid or peptide, an anti-inflammatory agent, an acne therapeutic agent, a microbicide, female hormone, a keratolytic agent and a natural product.

Examples of moisturizer include, but are not limited to, creatine, polyglutamic acid, sodium lactate, hydroproline, 2-pyrrolidone-5-carboxyclic acid sodium salt, hyaluronic acid, sodium hyaluronate, ceramide, phytosteryl, cholesterol, sitosterol, pullulan and proteoglycan. Examples of whitening agent include, but are not limited to, arbutin and a derivative thereof, kojic acid, bisabolol, niacinamide, vitamin C and a derivative thereof, placenta, allantoin and madecassoside. Examples of anti-wrinkle agent include, but are not limited to, retinol, retinol derivative, retinal, adenosine, licorice extract, red ginseng extract and ginseng extract. Examples of UV blocking agent include, but are not limited to, benzophenone derivative, para-aminobenzoic acid derivative, methoxycinnamic acid derivative, salicylic acid derivative and avobenzone. There is no special limitation to a hair growth promoter, but it may be preferably a blood circulation promoter and/or a hair follicle stimulant. Examples of blood circulation promoter include, but are not limited to, the extract of *Swertia japonica* Makino, cepharanthin, vitamin E and a derivative thereof and gamma-oryzanol, and examples of hair follicle stimulant include, but are not limited to, capsicum tincture, ginger tincture, cantharides tincture and nicotinic acid benzyl ester. Examples of vitamin or a derivative thereof include, but are not limited to, vitamin A (retinol) and a derivative thereof, vitamin B1, vitamin B2, vitamin B6, vitamin E and derivatives thereof, vitamin D, vitamin H, vitamin K, pantothenic acid and derivatives thereof, biotin, panthenol, coenzyme Q₁₀ and idebenone. Examples of amino acid or peptide include, but are not limited to, cysteine, methionine, serine, lysine, tryptophan, amino acid extract, epidermal growth factor (EGF), insulin-like growth factor (IGF), fibroblast growth factor (FGF), copper peptide, copper tripeptide-1, tripeptide-29, tripeptide-1, acetyl hexapeptide-8, nicotinoyl tripeptide-35, hexapeptide-12, hexapeptide-9, palmitoyl pentapeptide-4, palmitoyl tetrapeptide-7, palmitoyl tripeptide-29, palmitoyl tripeptide-1, nonapeptide-7, tripeptide-10 citrulline, sh-polypeptide-15, palmitoyl tripeptide-5, diaminopropionoyl tripeptide-33 and r-spider polypeptide-1. Examples of anti-inflammatory agent include, but are not limited to, beta-glycyrrhetinic acid, glycyrrhetinic acid derivative, aminocaproic acid, hydrocortisone, β-glucan and licorice. Examples of acne therapeutic agent include, but are not limited to, estradiol, estrogen, ethinyl estradiol, triclosan and azelaic acid. Examples of microbicide include, but are not limited to, benzalkonium chloride, benzethonium chloride and halocalban. There is no special limitation to female hormone, but it may be preferably estrogen. As estrogen, it may be preferably estradiol, ethinyl estradiol or isoflavone which is a phytoestrogen. Examples of keratolytic agent include, but are not limited to, sulfur, salicylic acid, AHA, BHA and resorcin. Examples of the extract of natural product or an ingredient obtained therefrom include, but are not limited to, the extract of Japanese witch-hazel, *Lamium album* var. barbatum, *Hedyotis diffusa, Rheum palmatum,* licorice, aloe, chamomile, rose hip, horse chestnut, ginseng, *Luffa aegyptiaca,* cucumber, laver, sea mustard, *Dioscorea batatas,* snail, fruit of *Dioscorea polystachya* and *Centella asiatica,* or hinokitiol and beta-carotene. In addition, yeast extract, collagen, elastin, DHA, EPA, flavor ingredient and the like may be used.

The cosmetic composition of the present invention may further comprise ingredients such as a stabilizer, an antioxidant and a lubricant, if necessary.

### EFFECTS OF INVENTION

The hydrangea-shaped layered double hydroxide prepared according to the present invention can deliver cosmetic active ingredients into the skin very efficiently in a stable form by providing a large specific surface area.

### BRIEF DESCRIPTION OF DRAWINGS

Figure 1 is a schematic diagram schematically illustrating the preparation method of Example 1.
Figure 2 is a photograph showing a volume comparison of the hydrangea-shaped layered double hydroxide (HLDH) of the present invention and the conventional layered double hydroxide (PLDH).
Figure 3 shows the results of FT-IR analysis of the hydrangea-shaped layered double hydroxide (HLDH) of the present invention and the conventional layered double hydroxide (PLDH).
Figure 4 is scanning electron microscope photographs of the hydrangea-shaped layered double hydroxide (HLDH) of the present invention and the conventional layered double hydroxide (PLDH).
Figure 5 is scanning electron microscope photographs of the HLDH comprising avobenzone of Example 2 and the PLDH comprising avobenzone of Comparative Example 1.
Figure 6 shows the results of FT-IR analysis of the HLDH comprising avobenzone of Example 2 and the PLDH comprising avobenzone of Comparative Example 1.
Figure 7 shows the HPLC analysis results of the HLDH comprising avobenzone in Example 2 and the PLDH comprising avobenzone in Comparative Example 1.
Figure 8 shows the results of particle distribution and zeta potential measurements in Experimental Example 3.
Figure 9 is a graph showing the results of measuring the degree of improvement in skin moisture content in Experimental Example 5.
Figure 10 is a graph showing the improvement rate in skin moisture content measured in Experimental Example 5.
Figure 11 is a graph showing the results of measuring the degree of improvement in desquamation index in Experimental Example 6.
Figure 12 is a graph showing the rate of improvement in desquamation index measured in Experimental Example 6.

### MODE FOR THE INVENTION

Hereinafter, the present invention is explained in more detail with the following examples. However, it must be understood that the protection scope of the present invention is not limited to the examples.

### Example 1: Preparation of hydrangea-shaped layered double hydroxide

A carbonate ion-containing layered double hydroxide was synthesized by a co-precipitation method as follows. A mixed solution (A) in which 1.875 M Mg(NO₃)₂·6H₂O (Duksan Science, Korea) and 0.625 M Al(NO₃)₃·9H₂O (Duksan Science, Korea) were dissolved in distilled water, and a solution (B) containing 4 M NaOH (Duksan Science, Korea) were slowly added to 0.5 M Na₂CO₃ (Duksan Science, Korea) solution (C) using pumps, respectively. The pH of the mixed solution (C) was maintained at 10.0 ± 0.5 during the addition. The mixed solution was stirred for approximately 1 hour, filtered using a Buchner funnel, washed with distilled water until the pH reached 7, and then washed by the use of ethanol. The clay collected on top of the Buchner funnel was placed back into ethanol and stirred for one day. After filtering using a Buchner funnel, the obtained product was vacuum-dried at room temperature using a vacuum chamber to obtain a hydrangea-shaped layered double hydroxide (hereinafter referred to as "HLDH"). The formula of the synthesized HLDH is as follows: [Mg₃Al(OH)₈]CO₃·xH₂O]

For comparison with the above HLDH, a conventional layered double hydroxide, Mg₄Al₂(OH)₁₂CO₃·3H₂O (plate-shaped layered double hydroxide, hereinafter referred to as "PLDH"), was purchased from Dansuk Co., Ltd. (Korea).

### Example 2: Preparation of HLDH comprising avobenzone

6 g of PARSOL^{®} 1789 (avobenzone) was dispersed in an aqueous solution of 1:1 ratio of water and ethanol, and 50 mL of distilled water in which 1.5 g of sodium hydroxide was dissolved was added thereto, followed by stirring at room temperature for 30 minutes. 10 g of HLDH was added to the mixed solution, stirred at 80°C for 5 hours, and then washed and filtered with hot distilled water and ethanol using a Buchner funnel. The clay collected on top of the Buchner funnel was vacuum-dried by using a vacuum chamber at room temperature.

### Comparative Example 1: Preparation of PLDH comprising avobenzone

PLDH comprising avobenzone was prepared in the same manner as in Example 2, except that PLDH was used instead of HLDH.

### Example 3: Preparation of HLDH comprising collagen

10 g of collagen was dispersed in water and stirred for 30 minutes. 10 g of HLDH was added to the mixed solution, stirred at room temperature for 24 hours, and then washed and filtered with distilled water and ethanol using a centrifuge. The clay collected at the bottom was vacuum-dried by using a vacuum chamber at room temperature.

### Example 4: Preparation of HLDH comprising hyaluronic acid

0.5 g of hyaluronic acid was dispersed in water and stirred for 30 minutes. 10 g of HLDH was added to the mixed solution, stirred at room temperature for 24 hours, and then washed and filtered with distilled water and ethanol using a centrifuge. The clay collected at the bottom was vacuum-dried by using a vacuum chamber at room temperature.

### Example 5: Preparation of HLDH comprising ceramide

1.5 g of ceramide was dispersed in an aqueous solution of 1:1 ratio of water and ethanol, and stirred for 30 minutes. 10 g of HLDH was added to the mixed solution, stirred at room temperature for 24 hours, and then washed and filtered with distilled water and ethanol using a centrifuge. The clay collected at the bottom was vacuum-dried by using a vacuum chamber at room temperature.

### Example 6: Preparation of HLDH comprising retinol

4.5 g of retinol was dispersed in an aqueous solution of 1:1 ratio of water and ethanol, and stirred for 30 minutes. 10 g of HLDH was added to the mixed solution, stirred at room temperature for 24 hours, and then washed and filtered with distilled water and ethanol using a centrifuge. The clay collected at the bottom was vacuum-dried by using a vacuum chamber at room temperature.

### Example 7: Preparation of HLDH comprising retinal

5 g of retinal was dispersed in an aqueous solution of 1:1 ratio of water and ethanol, and stirred for 30 minutes. 10 g of HLDH was added to the mixed solution, stirred at room temperature for 24 hours, and then washed and filtered with distilled water and ethanol using a centrifuge. The clay collected at the bottom was vacuum-dried by using a vacuum chamber at room temperature.

### Example 8: Preparation of HLDH comprising arbutin

5 g of arbutin was dispersed in an aqueous solution of 1:1 ratio of water and ethanol, and stirred for 30 minutes. 10 g of HLDH was added to the mixed solution, stirred at room temperature for 24 hours, and then washed and filtered with distilled water and ethanol using a centrifuge. The clay collected at the bottom was vacuum-dried by using a vacuum chamber at room temperature.

### Example 9: Preparation of HLDH comprising tocopherol

5 g of tocopherol was dispersed in an aqueous solution of 1:1 ratio of water and ethanol, and stirred for 30 minutes. 10 g of HLDH was added to the mixed solution, stirred at room temperature for 24 hours, and then washed and filtered with distilled water and ethanol using a centrifuge. The clay collected at the bottom was vacuum-dried by using a vacuum chamber at room temperature.

### Example 10: Preparation of HLDH comprising EGF

5 g of epidermal growth factor (EGF) was dispersed in water and stirred for 30 minutes. 10 g of HLDH was added to the mixed solution, stirred at room temperature for 24 hours, and then washed and filtered with distilled water and ethanol using a centrifuge. The clay collected at the bottom was vacuum-dried by using a vacuum chamber at room temperature.

### Example 11: Preparation of HLDH comprising copper peptide

5 g of copper peptide was dispersed in water and stirred for 30 minutes. 10 g of HLDH was added to the mixed solution, stirred at room temperature for 24 hours, and then washed and filtered with distilled water and ethanol using a centrifuge. The clay collected at the bottom was vacuum-dried by using a vacuum chamber at room temperature.

### Example 12: Preparation of HLDH comprising β-glucan

5 g of β-glucan was dispersed in water and stirred for 30 minutes. 10 g of HLDH was added to the mixed solution, stirred at room temperature for 24 hours, and then washed and filtered with distilled water and ethanol using a centrifuge. The clay collected at the bottom was vacuum-dried by using a vacuum chamber at room temperature.

### Example 13: Preparation of HLDH comprising isoflavone

5 g of isoflavone was dispersed in an aqueous solution of 1:1 ratio of water and ethanol, and stirred for 30 minutes. 10 g of HLDH was added to the mixed solution, stirred at room temperature for 24 hours, and then washed and filtered with distilled water and ethanol using a centrifuge. The clay collected at the bottom was vacuum-dried by using a vacuum chamber at room temperature.

### Example 14: Preparation of HLDH comprising salicylic acid

5 g of salicylic acid was dispersed in an aqueous solution of 1:1 ratio of water and ethanol, and stirred for 30 minutes. 10 g of HLDH was added to the mixed solution, stirred at room temperature for 24 hours, and then washed and filtered with distilled water and ethanol using a centrifuge. The clay collected at the bottom was vacuum-dried by using a vacuum chamber at room temperature.

### Example 15: Preparation of HLDH comprising Centella asiatica extract

5 g of *Centella asiatica* extract was dispersed in water and stirred for 30 minutes. 10 g of HLDH was added to the mixed solution, stirred at room temperature for 24 hours, and then washed and filtered with distilled water and ethanol using a centrifuge. The clay collected at the bottom was vacuum-dried by using a vacuum chamber at room temperature.

### Example 16: Preparation of HLDH comprising madecassoside

5 g of madecassoside was dispersed in an aqueous solution of 1:1 ratio of water and ethanol, and stirred for 30 minutes. 10 g of HLDH was added to the mixed solution, stirred at room temperature for 24 hours, and then washed and filtered with distilled water and ethanol using a centrifuge. The clay collected at the bottom was vacuum-dried by using a vacuum chamber at room temperature.

### Example 17 and Comparative Example 2: Preparation of creams containing avobenzone

The ingredients of phases A, B-1, B-2 and C in Table 1 were mixed respectively. Phases A and B-2 were mixed at 75°C or higher. After the ingredients were completely dissolved, they were mixed using a homo mixer for a certain period of time and cooled to prepare a cream.

**[Table 1]**

| | Ingredient | Example 17 | Comparative Example 2 |
|---|---|---|---|
| A | Lanette-O (Kalcol 6850) | 0.80 | 0.80 |
| | Stearic acid | 0.60 | 0.60 |
| | TEGO CARE #450 (emulsifier) | 3.00 | 3.00 |
| | Macadamia nut oil | 6.00 | 6.00 |
| | CEH (cetyl ethylhexanoate) | 4.00 | 4.00 |
| | Neo-squalane | 4.00 | 4.00 |
| | 6cs (sera sense 6SF) | 0.50 | 0.50 |
| | GMS 105 (glyceryl monostearate) | 0.50 | 0.50 |
| B-1 | EDTA-2Na | 0.01 | 0.01 |
| | Water (hot) | 47.19 | 50.69 |
| | Glycerin | 3.00 | 3.00 |
| | Carbopol #940 (2.5% soln) | 16.00 | 16.00 |
| | Polyglutamic acid | 0.05 | 0.05 |
| B-2 | 1,2-Hexanediol | 2.00 | 2.00 |
| | 1,3-Butylene glycol | 5.95 | 5.95 |
| C | TEA (triethanolamine) | 0.40 | 0.40 |
| | Water | 1.00 | 1.00 |
| D | HLDH of Example 2 (containing 30 wt% avobenzone) | 5.00 | - |
| | Avobenzone | - | 1.50 |

| | | | |
|---|---|---|---|
| (unit: g) | | | |

### Example 18 and Comparative Example 3: Preparation of creams containing hyaluronic acid

The ingredients of phases A, B-1, B-2 and C in Table 2 were mixed respectively. Phases A and B-2 were mixed at 75°C or higher. After the ingredients were completely dissolved, they were mixed using a homo mixer for a certain period of time and cooled to prepare a cream.

**[Table 2]**

| | Ingredient | Example 18 | Comparative Example 3 |
|---|---|---|---|
| A | Cetostearyl alcohol | 0.80 | 0.80 |
| | Stearic acid | 0.60 | 0.60 |
| | Polyglyceryl-3 methylglucose distearate | 3.00 | 3.00 |
| | Macadamia nut oil | 6.00 | 6.00 |
| | Cetylethylhexanoate | 4.00 | 4.00 |
| | Squalane | 4.00 | 4.00 |
| | Dimethicone | 0.50 | 0.50 |
| B-1 | EDTA-2Na | 0.01 | 0.01 |
| | Water (hot) | 54.69 | 50.69 |
| | Glycerin | 3.00 | 3.00 |
| | Carbopol #940 (2.5% soln) | 16.00 | 16.00 |
| B-2 | 1,2-Hexanediol | 2.00 | 2.00 |
| | 1,3-Butylene glycol | 3.00 | 3.00 |
| C | TEA (triethanolamine) | 0.40 | 0.40 |
| | Water | 1.00 | 1.00 |
| D | HLDH of Example 4 (containing 5 wt% hyaluronic acid) | 1.00 | - |
| | 1 wt% hyaluronic acid solution | - | 5.00 |

| | | | |
|---|---|---|---|
| (unit: g) | | | |

### Example 19 and Comparative Example 4: Preparation of creams containing retinol

The ingredients of phases A, B-1, B-2 and C in Table 3 were mixed respectively. Phases A and B-2 were mixed at 75°C or higher. After the ingredients were completely dissolved, they were mixed using a homo mixer for a certain period of time and cooled to prepare a cream.

**[Table 3]**

| | Ingredient | Example 19 | Comparative Example 4 |
|---|---|---|---|
| A | Cetostearyl alcohol | 0.80 | 0.80 |
| | Stearic acid | 0.60 | 0.60 |
| | Polyglyceryl-3 methylglucose distearate | 3.00 | 3.00 |
| | Macadamia nut oil | 6.00 | 6.00 |
| | Cetylethylhexanoate | 4.00 | 4.00 |
| | Squalane | 4.00 | 4.00 |
| | Dimethicone | 0.50 | 0.50 |
| B-1 | EDTA-2Na | 0.01 | 0.01 |
| | Water (hot) | 53.19 | 55.44 |
| | Glycerin | 3.00 | 3.00 |
| | Carbopol #940 (2.5% soln) | 16.00 | 16.00 |
| B-2 | Hexanediol | 2.00 | 2.00 |
| | 1,3-Butylene glycol | 3.00 | 3.00 |
| C | TEA (triethanolamine) | 0.40 | 0.40 |
| | Water | 1.00 | 1.00 |
| D | HLDH of Example 6 (containing 10 wt% retinol) | 2.50 | - |
| | Retinol | - | 0.25 |

| | | | |
|---|---|---|---|
| (unit: g) | | | |

### Experimental Example 1: Analysis of LDH

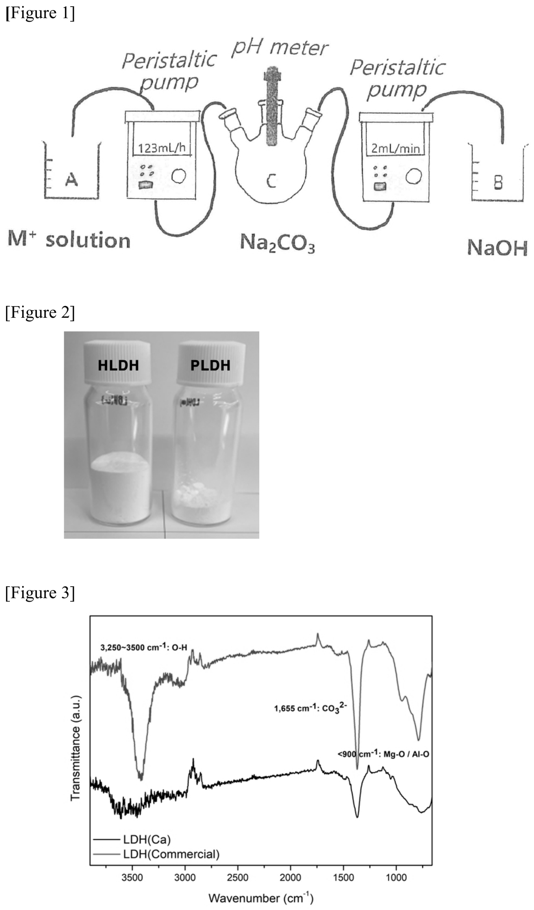

When the PLDH and the HLDH of the present invention were measured by BET, the PLDH had a specific surface area of 150 m²/g, while the HLDH had a specific surface area of 285.58 m²/g. This result confirmed that the HLDH-which has a hydrangea-like shape-has a larger specific surface area. In addition, as a volume comparison experiment, the same amount of 2 g was placed in the same vial, and it was confirmed that the HLDH has 4 times higher volume (Figure 2).

The structure of the synthesized HLDH was confirmed by identifying chemical functional groups through Fourier-transform infrared spectroscopy (FT-IR) analysis (Figure 3). The broad absorption band observed in the 3,250-3,500 cm⁻¹ region is attributed to the hydroxy group (O-H) in the PLDH, and the strong absorption band at 1,655 cm⁻¹ is attributed to the carbonate anion (CO₃²⁻). Absorption bands appearing below 900 cm⁻¹ are attributed to the bond of aluminum and oxygen, and magnesium and oxygen.

In addition, the shape and size of the PLDH and the HLDH of the present invention were examined using a scanning electron microscope (SEM) (Figure 4). The PLDH exhibited a plate-like structure, while the HLDH had a hydrangea-like shape. This supports the results of the BET analysis that the HLDH has a wider specific surface area.

### Experimental Example 2: Analysis of LDH containing avobenzone

FT-IR analysis was performed to analyze the structures of the HLDH containing avobenzone of Example 2 and the PLDH containing avobenzone of Comparative Example 1. Absorption bands were observed at 3,250-3,500 cm⁻¹, 1,655 cm⁻¹ and 900 cm⁻¹, respectively. Additionally, absorption bands of PARSOL^{®} 1789 (avobenzone) were observed: a carbon-hydrogen bond (C-H) at 2,970 cm⁻¹, a carbon-oxygen double bond (C=O) at 1,600 cm⁻¹ and a carbon-oxygen single bond (C-O) at 1,050-1,250 cm⁻¹.

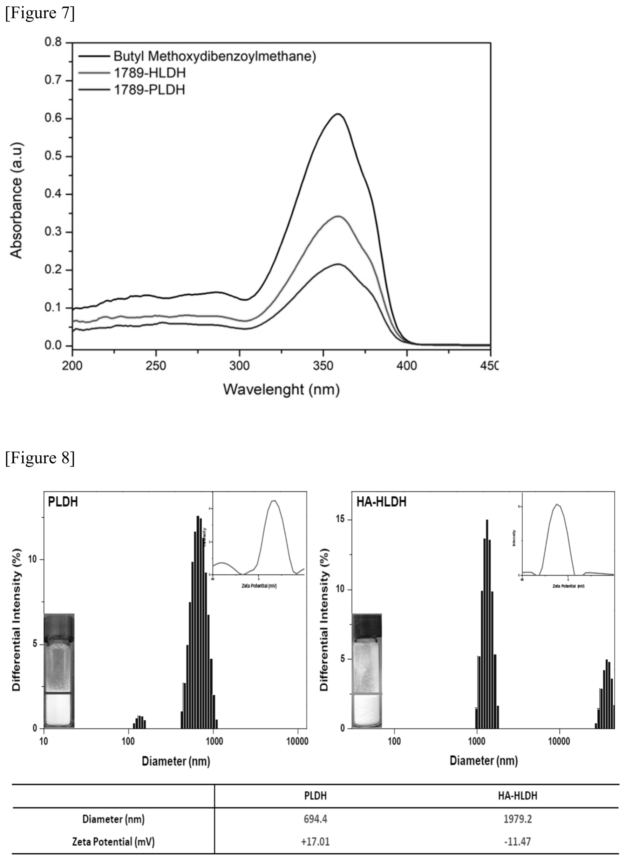

In addition, the shape and size of the HLDH of Example 2 and the PLDH of Comparative Example 1 were confirmed through SEM analysis (Figure 5). Specifically, in the case of the HLDH of Example 2, it was found that PARSOL^{®} 1789 was evenly captured between the hydrangea-shaped particles with a large specific surface area. Furthermore, to confirm whether the active ingredient, PARSOL^{®} 1789, was captured within the layered double hydroxide, analysis was performed using a UV-vis spectrophotometer using ethanol solvent (Figure 6). While the existing PARSOL^{®} 1789 exhibited a maximum peak value at 358 nm, both the HLDH of Example 2 and the PLDH of Comparative Example 1 were confirmed to have maximum peak values at 358 nm. The HLDH of Example 2 had a higher onset value, indicating a higher PARSOL^{®} 1789 content. High-performance liquid chromatography (HPLC) analysis was performed to quantitatively analyze the PARSOL^{®} 1789 content within the HLDH of Example 2 and the PLDH of Comparative Example 1 (Figure 7). In the case of the PLDH of Comparative Example 1, the content was 12.56%, while in the case of Example 2, which used the HLDH in the shape of hydrangea with a large specific surface area, the content was found to be about twice as high at 26.71%.

### Experimental Example 3: Measurement of particle distribution and zeta potential

The particle distribution and zeta potential of the PLDH and the HLDH containing hyaluronic acid of Example 4 were measured using a Photal ELS-Z (Photal, Japan). The average particle size was 694.4 nm for the PLDH and 1979.2 nm for the HLDH of Example 4. The zeta potential was +17.01 for the PLDH and -11.47 for the HLDH of Example 4 (Figure 8).

### Experimental Example 4: Measurement of UV protection effect

The UV protection effect was measured using creams containing avobenzone of Example 17 and Comparative Example 2. As a result, it was found that the cream containing the HLDH of the present invention of Example 17 had a higher PA index (protection grade of UVA) with less avobenzone compared to the cream of Comparative Example 2 in which avobenzone was added as is.

### Experimental Example 5: Measurement of moisturizing effect

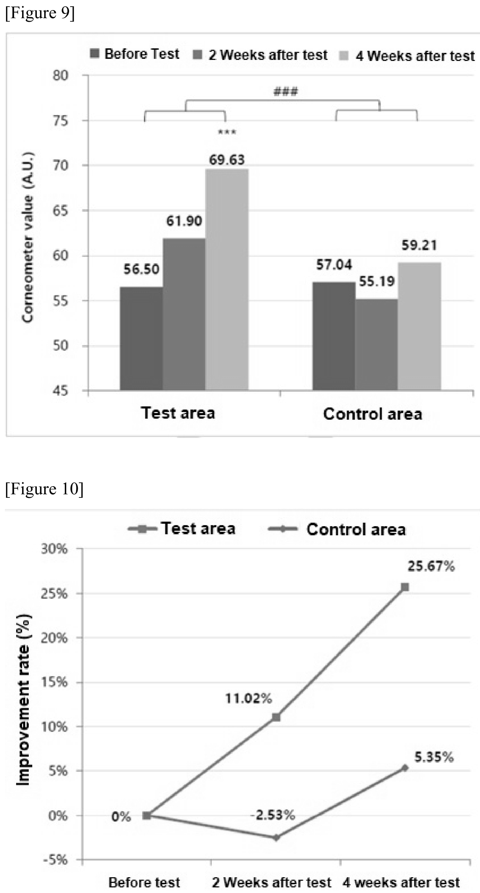

After applying the creams containing hyaluronic acid of Example 18 and Comparative Example 3, a probe attached to Corneometer^{®} CM 825 (Courage + Khazaka electronic GmbH, Germany) was placed vertically against the skin surface and gently pressed to measure moisture content. It was confirmed that the cream of Example 18 containing the HLDH of the present invention had a better moisturizing effect than the cream of Comparative Example 3 in which hyaluronic acid was added as is (Figures 9 and 10).

### Experimental Example 6: Measurement of stratum corneum improvement effect

After applying the creams containing hyaluronic acid of Example 18 and Comparative Example 3, stratum corneum samples were collected from the skin surface using Corneofix F20 (Courage + Khazaka electronic GmbH, Germany). The skin surface was analyzed using Visioscan VC98 (Courage + Khazaka electronic GmbH, Germany) to measure the stratum corneum improvement effect.

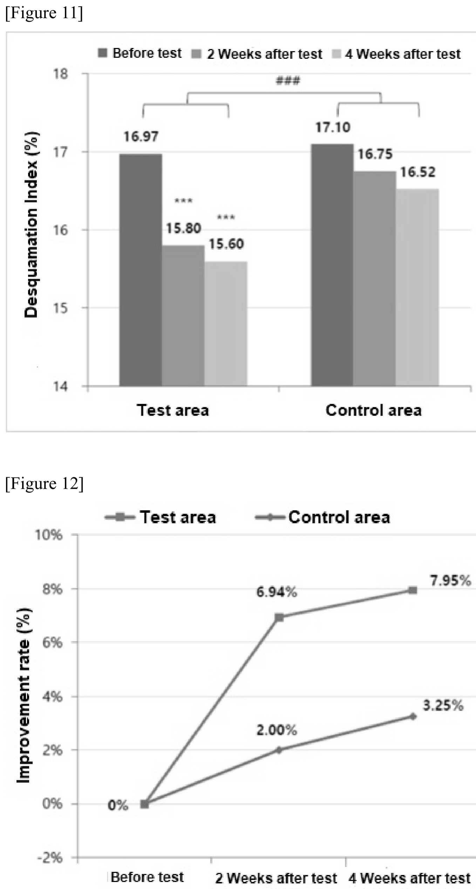

It was confirmed that the cream of Example 18 containing the HLDH of the present invention showed a better desquamation index than the cream of Comparative Example 3 in which hyaluronic acid was added as is (Figures 11 and 12).

### Experimental Example 7: Test for effect on promoting transdermal absorption

The artificial skin, Neoderm (Tego Science, Korea) was mounted to a Franz-type diffusion cell (Lab Fine Instruments, Korea). 50 mM phosphate buffer (pH 7.4, 0.1M NaCl) was added to a receptor cell (5 ml) of the Franz-type diffusion cell. A diffusion cell was then mixed and diffused at 600 rpm, 32°C, and 50 µl of the creams containing retinol of Example 19 and Comparative Example 4, respectively, were added to donor cells. Absorption and diffusion were carried out according to the predetermined time, and the area of the skin where the absorption and diffusion were carried out was 0.64 cm². After finishing the absorption and diffusion of the active ingredient, the residues-which were not absorbed and remained on the skin-were cleaned with dried Kimwipes^{™} or 10 ml of ethanol. The skin in which the active ingredient was absorbed and diffused was homogenized by the use of a tip-type homogenizer, and retinol absorbed into the skin was then extracted with 4 ml of dichloromethane. The extract was then filtrated with a 0.45 µm nylon membrane filter. The content of retinol was measured by high-performance liquid chromatography (HPLC) with the following conditions, and the results are represented in Table 4.

**[Table 4]**

| | Transdermal absorption (µg) | Rate of increase |
|---|---|---|
| Cream of Example 19 | 0.354 | 19.03% |
| Cream of Comparative Example 4 | 0.186 | - |
| A) Column: C18 (4.6 × 150 mm, 5 µm) | | |
| B) Mobile phase: methanol | | |
| C) Flow rate: 1.0 mL/min | | |
| D) Detector: UV 325 nm | | |

## Claims

1. A method for preparing a hydrangea-shaped layered double hydroxide comprising:
i) adding a mixed solution of a solution comprising a divalent metal cation and a solution comprising a trivalent metal cation, and a NaOH solution to a solution comprising an anion while maintaining pH at 9 to 11;
ii) stirring the mixed solution obtained in step (i);
iii) filtering the resultant obtained in step (ii) through a Buchner funnel and washing with distilled water until pH reaches 6 to 8;
iv) adding a solvent to the resultant collected at the top of the Buchner funnel obtained in step (iii) and stirring; and
v) filtering the resultant obtained in step (iv) through a Buchner funnel and drying.

2. The method for preparing a hydrangea-shaped layered double hydroxide according to Claim 1, wherein the divalent metal cation of step (i) is Ca²⁺, Mg²⁺, Zn²⁺, Ni²⁺, Mn²⁺, Co²⁺ or Fe²⁺.

3. The method for preparing a hydrangea-shaped layered double hydroxide according to Claim 1, wherein the trivalent metal cation of step (i) is Al³⁺, Cr³⁺, Mn³⁺, Fe³⁺, Ga³⁺, Co³⁺ or Ni³⁺.

4. The method for preparing a hydrangea-shaped layered double hydroxide according to Claim 1, wherein the anion of step (i) is OH⁻, F⁻, Cl⁻, Br⁻, I⁻, NO₃⁻, CO₃²⁻ or SO₄²-.

5. The method for preparing a hydrangea-shaped layered double hydroxide according to Claim 1, wherein 2 to 5M NaOH is used in step (i).

6. The method for preparing a hydrangea-shaped layered double hydroxide according to Claim 1, wherein the pH of the solution comprising the anion is maintained at 9.2 to 10.8 in step (i).

7. The method for preparing a hydrangea-shaped layered double hydroxide according to Claim 6, wherein the pH of the solution comprising the anion is maintained at 10 + 0.5 in step (i).

8. The method for preparing a hydrangea-shaped layered double hydroxide according to Claim 1, wherein the mixed solution of the solution comprising the divalent metal cation and the solution comprising the trivalent metal cation, and the NaOH solution is added to the solution comprising the anion at a rate of 1 to 4 mL/min in step (i).

9. The method for preparing a hydrangea-shaped layered double hydroxide according to Claim 1, wherein the mixed solution is stirred for 30 minutes to 2 hours in step (ii).

10. The method for preparing a hydrangea-shaped layered double hydroxide according to Claim 1, wherein the step (iii) further comprises washing with ethanol.

11. The method for preparing a hydrangea-shaped layered double hydroxide according to Claim 1, wherein the solvent of step (iv) is ethanol.

12. The method for preparing a hydrangea-shaped layered double hydroxide according to Claim 1, wherein the stirring is carried out for 12 to 36 hours in step (iv).

13. The method for preparing a hydrangea-shaped layered double hydroxide according to Claim 1, wherein the drying in step (v) is carried out by using a vacuum chamber at room temperature.

14. The method for preparing a hydrangea-shaped layered double hydroxide according to Claim 1, the hydrangea-shaped layered double hydroxide has a BET surface area of 200 m²/g or more.

15. A cosmetic composition comprising a hydrangea-shaped layered double hydroxide prepared by a method as defined in any one of Claims 1 to 14, and an active ingredient.

16. The cosmetic composition according to Claim 15, wherein the active ingredient is selected from the group consisting of a moisturizer, a whitening agent, an anti-wrinkle agent, a UV blocking agent, a hair growth promoter, vitamin or a derivative thereof, amino acid or peptide, an anti-inflammatory agent, an acne therapeutic agent, a microbicide, female hormone, a keratolytic agent and a natural product.
